# EUROPÄISCHE PATENTSCHRIFT

(11) **EP 2 295 001 B1**
(45) Veröffentlichungstag und Bekanntmachung des Hinweises auf die Patenterteilung: **09.03.2016**
(21) Anmeldenummer: 10009317.8
(22) Anmeldetag: 08.09.2010
(51) Int. Cl.: A61B 50/00

(54) **Einwegbehälter zum Darbieten von Verbandstoffen sowie wegwerfbares OP-Spendersystem**
Disposable container for dressings and disposable OP dispenser system
Récipient à une voie pour mettre à disposition des pansements et système de distribution OP jetable

(30) Priorität: 15.09.2009 DE 102009041529
(43) Veröffentlichungstag der Anmeldung: 16.03.2011
(73) Patentinhaber: Paul Hartmann Aktiengesellschaft, 89522 Heidenheim (DE)
(72) Erfinder: Disch, Karl-Heinz, 79215 Elzach (DE); Krompholz, Michael, 78112 St. Georgen (DE)
(74) Vertreter: DREISS Patentanwälte PartG mbB

(56) Entgegenhaltungen:
- WO-A2-2005/062996
- US-A- 3 628 692
- US-A1- 2008 000 910

## Beschreibung

Die Erfindung betrifft einen Einwegbehälter zum Darbieten von Verbandstoffen im Operationssaal gemäss Anspruch 1.

Es ist im Stand der Technik bekannt, im Bereich von Operationssälen und Räumlichkeiten die benötigten Verbandstoffe vor einer Operation in Operationsmetallbehälter einzufüllen, zu verpacken und zu sterilisieren. Dies wird vom Krankenhauspersonal zur Vorbereitung einer Operation vorgenommen. Diese Metallbehälter können dann über an ihnen vorgesehene Winkel direkt am Instrumententisch des Operationssaals eingehängt werden. Alternativ sind Verbandstoffbehälter bekannt, die auf dem Instrumententisch abgestellt werden. Dies bedingt jedoch den Nachteil, dass auf dem Instrumententisch weniger Platz für die benötigten chirurgischen Instrumente zur Verfügung steht.

Nachteilig bei den bisherigen Operationsbehältnissen ist dabei, dass die Behälter im Krankenhaus einzeln zusammengestellt, verpackt und sterilisiert werden müssen und der Sterilisationsprozess überwacht und validiert werden muss, sofern es sich um derartige einhängbare mehrfach verwendbare Metallbehälter handelt.

Sofern in der Vergangenheit bereits Einwegbehälter verwendet wurden, besteht der Nachteil, dass diese entweder zusätzlich in den entsprechenden Metallbehältern angeordnet werden müssen, was einen zusätzlichen Sterilisationsschritt bedingt, oder auf dem Instrumententisch bereitgestellt werden müssen, wodurch der zur Verfügung stehende Platz vermindert wird.

Aus der WO 2005/1062996 A2 ist ein an die Bedürfnisse eines Benutzers anpassbares System aus mehreren Modulen bekannt, in denen kleinteilige Wundversorgungsprodukte aufgenommen sind, wobei die Module verbindbar und gegebenenfalls über einen Deckel wieder verschließbar verschlossen sind. Insbesondere muss der Deckel jedoch, wenn z. B. während einer Operation, auf die Materialien zugegriffen werden soll, jedes Mal bewegt werden und ist dem direkten Zugriff auf die Produkte im Weg.

Ausgehend von diesem Stand der Technik soll nun ein Einwegbehälter zum Darbieten von Verbandstoffen im Operationssaal bereitgestellt werden, der die vorstehenden Nachteile vermeidet.

Die Erfindung löst diese Aufgabe durch einen Einwegbehälter zum Darbieten von Verbandstoffen im Operationssaal mit den Merkmalen des Anspruchs 1. Darüber hinaus wird die Aufgabe durch ein wegwerfbares OP-Spendersystem umfassend einen Einwegbehälter nach einem der Ansprüche 1 bis 8 gelöst, wobei im Innenraum des Einwegbehälters herstellerseitig sterile, absorbierende Verbandstoffe vorgelegt sind.

Dabei ist erfindungsgemäß vorgesehen, dass mindestens ein Bereich des Deckels als Fixiermittelzone mit einem Fixiermittel ausgebildet ist, über das der Einwegbehälter an einer vertikalen und/oder horizontalen Fläche, insbesondere eines Instrumententischs, fixierbar ist. Damit werden die Vorteile,
die bisher bereits bei Mehrwegbehältnissen bestanden, nämlich der zusätzliche Platzgewinn auf dem Instrumententisch in Operationssälen auch für Einwegbehältnisse verfügbar gemacht, die bisher auf dem Instrumententisch selber abgestellt werden mussten. Durch die Vorsehung einer Fixiermittelzone mit einem Fixiermittel im Bereich des Deckels kann der Einwegbehälter an einer horizontalen und/oder vertikalen Fläche derart angelenkt werden, dass er ebenfalls zur Vergrößerung der zur Verfügung stehenden Fläche, insbesondere des Instrumententischs insoweit beiträgt, als dass keine Fläche auf dem Instrumententisch für die Aufstellung des Einwegbehälters benötigt wird. Zusätzlich kann vorgesehen sein, dass mindestens ein Bereich mindestens einer Seitenfläche als Fixiermittelzone mit einem Fixiermittel ausgebildet ist, über das der Einwegbehälter an einer vertikalen und/oder horizontalen Fläche, insbesondere des Instrumententisches, fixiert ist.

Dass ein Abschnitt mindestens einer der mindestens einen Seitenflächen in einer Fixiermittelzone ein Fixiermittel aufweist, besitzt den Vorteil, dass die Seitenflächen des Einwegbehälters an einer vertikalen Fläche, insbesondere an einer vertikalen Fläche des Instrumententisches über das Fixiermittel festgelegt werden können. In aller Regel weist ein Instrumententisch für einen Operationsraum dabei eine horizontale nach oben gerichtete Oberfläche mit vier Seitenflächen auf, so dass eine Festlegung des Einwegbehälters an einer der Seitenflächen des Instrumententisches mittels des Fixermittels in der Fixiermittelzone erfolgen kann.

Zusätzlich kann das Fixiermittel auch derart vorgesehen sein, dass es sich über die an der mindestens einen Seitenfläche des Einwegbehälters vorgesehenen Fixiermittelzone hinaus erstreckt, und damit vorteilhaft in Richtung auf eine horizontale Fläche, insbesondere auf eine horizontale Fläche eines Instrumententisches umgelegt und auf dieser horizontalen Fläche befestigt werden kann. Alternativ kann auch vorgesehen sein, sofern ein fest mit dem Behälter verbundener Deckel an diesem angelenkt ist, dass die Unter- bzw. Innen- oder Ober- bzw. Außenseite des Deckels, also die auf den Innenraum bei geschlossenem Deckel hin gerichtete Seite oder die davon abgewandte Seite, mit einer Fixiermittelzone versehen sind. Alternativ können auch beide Seiten des Deckels eine Fixiermittelzone aufweisen, wobei dann entweder der Deckel so weit geöffnet werden kann, dass er mit seiner Außenseite, also der Oberseite, gegen die Seitenwand des Behälters anliegt und dann mit dem auf der Innenseite angeordneten Fixiermittel ebenfalls an der vertikalen Fläche des Instrumententisches befestigt werden kann. Alternativ kann der Deckel auch um lediglich 180° aus der geschlossenen Position in eine geöffnete Position verschwenkt werden und dann über das Fixiermittel der Fixiermittelzone auf der Außenseite des Deckels mit der horizontalen Fläche des Instrumententisches verbunden werden. Dabei kann dann die Seitenfläche des Einwegbehälters gegen die vertikale Seitenfläche des Instrumententisches abgestützt werden, wodurch eine vergleichsweise stabile Befestigung erfolgen kann. Alternativ kann das Fixiermittel auch derart vorgesehen sein, dass es sich über die an der Unter- und/oder Außenseite des Deckels vorgesehenen Fixiermittelzone hinaus erstreckt, und damit vorteilhaft in Richtung auf eine horizontale Fläche, insbesondere auf eine horizontale Fläche eines Instrumententisches umgelegt und auf dieser horizontalen Fläche befestigt werden kann.

Der Einwegbehälter ist mit Deckel ausgebildet.

Der Deckel für den Einwegbehälter kann dabei in alternativen Ausführungsformen gestaltet sein.

Der Deckel kann zum einen unlösbar mit dem Einwegbehälter verbunden sein. Der Deckel ist erfindungsgemäß an dem Einwegbehälter über ein Gelenk angelenkt über eine die eine Seitenfläche des Einwegbehälters und den Deckel verbindende Kante.

Dabei kann vorgesehen sein, dass die verschlossenen Einwegbehälter nach dem Befüllen, vorzugsweise mit absorbierenden Verbandstoffen, und Verschließen durch den Hersteller in eine weitere Umverpackung als Sterilverpackung eingeführt und darin sterilisiert werden.

Besonders vorteilhaft ist darüber hinaus, wenn bei der Befüllung der Einwegbehälter diese so erfolgt, dass ein einfaches Zählen der Produkte, insbesondere der absorbierenden Verbandstoffe möglich ist. Dies kann beispielsweise durch eine entsprechende Positionierung der Produkte, z. B. mit den Faltkanten nach oben in Richtung Öffnung des Einwegbehälters bzw. Deckel, erreicht werden. Hierdurch wird dem Operationspersonal ein guter Überblick über die dargebotenen Absorptionsmaterialien geboten.

Darüber hinaus können durch die Bereitstellung derartiger wegwerfbarer Einwegbehälter, die mit absorbierenden Materialien herstellerseitig bereits befüllt sind, und insbesondere ein Basis-Sortiment an absorbierenden Materialien und/oder auch eine indikationsbezogene Zusammenstellung an absorbierenden Materialien aufweisen, die meisten Operationssituationen abgedeckt werden, so dass eine Einzelbefüllung durch das Krankenhauspersonal mit einzelner Sterilisation nicht mehr notwendig ist.

Bei dem Material des Einwegbehälters kann es sich insbesondere um ein Vollpappematerial handeln, insbesondere mit einem Flächengewicht von 250 bis 800 g/m², vorzugsweise 350 bis 600 g/m². Grundsätzlich ist auch ein Einsatz von Wellpappe möglich. Unter Wellpappe ist ein Mehrlagenverbund an Papier und/oder Pappe, aus zumindest einer ebenen Lage und einer daran festgelegten S-förmigen, eben einer gewellten Lage, zu verstehen. An die S-förmige Lage kann eine ebene Lage anschließen. Wellpappen mit weiteren Lagen sind denkbar.

Insbesondere können die Einwegbehälter als Faltschachteln ausgebildet sein. Weiter insbesondere können einzelne Kanten des Einwegbehälters, insbesondere der Faltschachteln verstärkt sein, um ein Ausbauchen durch das Gewicht der absorbierenden Produkte, aber auch während der Sterilisation der Produkte und der Einwegbehälter zu verhindern.

Der Einwegbehälter weist erfindungsgemäß mindestens eine Seitenfläche auf. Als Seitenfläche wird hier jegliche, eine Seitenwand des Einwegbehälters bildende Fläche verstanden. Im Falle einer kontinuierlichen, in sich geschlossenen und ohne durch eine Kante unterbrochene Seitenwand, liegt nur eine Seitenfläche vor.

Es ist auch denkbar, dass der Einwegbehälter drei Seitenflächen aufweist; es sind also in der Seitenwand drei Kanten vorhanden.

Insbesondere bevorzugt weist der Einwegbehälter vier Seitenflächen auf, d.h. die Seitenwand des Einwegbehälters weist entsprechend auch vier Kanten auf.

Insbesondere kann es sich bei den Einwegbehältern um quaderförmige Einwegbehälter handeln, bei dem die vier, insbesondere geraden Seitenflächen insbesondere zwei größere parallel beabstandete Längsflächen und zwei kleinere parallel beabstandete Längsflächen aufweisen. Die Größe, insbesondere die Länge der größeren Längsflächen kann dabei durch die Abmessungen des Instrumententisches, insbesondere der Länge einer Seitenfläche des Instrumententisches bestimmt sein. Die Breite und damit die Länge der kürzeren Seitenflächen sowie die Höhe der Seitenflächen kann durch die gefalteten Maße der Inhaltsteile bestimmt werden. Insbesondere vorzugsweise weist die kürzere Seitenfläche des Einwegbehälters eine Breite und/oder eine Höhe von nicht größer als 25 cm, vorzugsweise nicht größer als 20 cm und insbesondere vorzugsweise nicht größer als 15 cm auf. Auf diese Weise wird erreicht, dass der Einwegbehälter nicht zu weit über den Instrumententisch bzw. die Fläche, an der er angebracht ist, hinaussteht und so zu Behinderungen beiträgt. Darüber hinaus kann dadurch auch erreicht werden, dass der Einwegbehälter selber nicht zu schwer wird und somit ein sicheres Anhaften über das Fixiermittel an der Fläche, an der er befestigt ist, realisiert werden kann. Um ein vorteilhaftes Volumen bereitzustellen, soll die Breite und/oder Höhe der insbesondere kürzeren Seitenflächen mindestens 5 cm, weiter insbesondere mindestens 6 cm, weiter insbesondere mindestens 7 cm aufweisen.

Die Länge der längeren Seitenflächen beträgt vorzugsweise mindestens 20 cm, insbesondere mindestens 25 cm, weiter insbesondere mindestens 30 cm, weiter insbesondere mindestens 35 cm. Vorzugsweise beträgt die Länge der längeren Seitenflächen höchstens 70 cm, insbesondere höchstens 65 cm, weiter insbesondere höchstens 60 cm.

Besonders bevorzugt weist der Einwegbehälter dabei die Maße Länge x Breite x Höhe von 50 bis 55 cm x 7 bis 12 cm x 9 bis 10 cm auf. Die Abmessungen können dabei so angepasst sein, dass eine optimale Volumenauslastung während der Logistik und des Lagerungsprozesses gewährleistet ist.

Bevorzugt ist ein quaderförmiger Einwegbehälter mit vier, insbesondere geraden Seitenflächen vorgesehen. Alternativ kann auch vorgesehen sein, dass der Einwegbehälter eine gerade Seitenfläche aufweist, an der dann vorzugsweise auch die Fixiermittelzone angebracht ist, und der übrige Einwegbehälter eine gerundete Seitenfläche aufweist, die über zwei Kanten mit der geraden Seitenfläche verbunden ist. Hierdurch wird allerdings das Platzangebot begrenzt und die Verpackbarkeit von Absorptionsmaterialien beschränkt.

Besonders bevorzugt kann vorgesehen sein, dass sich das Fixiermittel an einer der Seitenflächen, vorzugsweise an einer geraden Seitenfläche, die ungekrümmt ausgebildet ist, befindet oder in einem Bereich einer Seitenfläche, der ungekrümmt ausgebildet ist.

Alternativ kann das Fixiermittel sich auch über die Fixiermittelzone und insbesondere die Seitenfläche und/oder den Deckel hinaus erstrecken. Die Fixierung des Einwegbehälters an der vertikalen und/der horizontalen Fläche kann dann im Bereich der Fixiermittelzone und/oder in überstehenden Bereichen des Fixiermittels erfolgen, die mit Flächen beispielsweise des Instrumententisches zusammenwirken.

Unter Fixiermittelzone ist dabei zumindest der Bereich zu verstehen, in welchem das jeweilige Fixiermittel an der Seitenfläche des Einwegbehälters und/oder an der Unter- und/oder Außenseite des Deckels unlösbar angeordnet ist. Das in der Fixiermittelzone befestigte Fixiermittel kann vorteilhafterweise bereits in diesem Bereich der Fixiermittelzone in seiner Verwendung zur Fixierung/Anhaftung an eine vertikale und/oder horizontale Fläche ausgebildet sein. Alternativ kann im Falle von Fixiermitteln, welche sich über die Fixiermittelzone hinauserstrecken, also über einen freien überhängenden, noch nicht gebundenen Bereich verfügen, die Fixermittelzone nicht für die Fixierung vorgesehen sein, sondern ausschließlich der freie Überhang, also der überstehende Bereich. Alternativ können auch sowohl der Bereich der mit der Fixiermittelzone verbunden ist, als auch der hierüber überstehende Bereich zur Fixierung an einer vertikalen und/oder horizontalen Fläche dienen.

Vorteilhafterweise weist das Fixiermittel einen freien Überhang mit einer derartigen Erstreckung auf, so dass sich das Fixiermittel über die obere Kante der Seitenfläche des Einwegbehälters und/oder über die freie Kante des Deckels hinauserstreckt. Dies bietet den Vorteil, dass das Fixiermittel je nach Bedarf noch umgelegt werden kann und so auf einer vertikalen und/oder horizontalen Fläche, insbesondere eines Instrumententisches befestigt werden kann. Hierdurch wird eine noch sicherere Befestigung erreicht.

Insbesondere kann die Fixiermittelzone an einer Seitenfläche im Bereich einer oberen Seitenkante einer Seitenfläche des Einwegbehälters angebracht sein.

Zusätzlich zu der Fixiermittelzone auf der Innen- und/oder Außenseite des Deckels kann die Fixiermittelzone an einer Seitenfläche des Einwegbehälters angebracht sein und so eine Fixierung, wie bereits vorstehend beschrieben, ermöglichen. Weiter vorzugsweise ist die Fixiermittelzone ausgehend von der mit dem Deckel verbundenen Seitenkante der Seitenfläche eher in Richtung der freien Seitenkante des Deckels angeordnet.

Die Breite bzw. Höhe der Fixiermittelzone, insbesondere eine an der Seitenfläche des Einwegbehälters angeordnete Fixiermittelzone ist vorzugsweise an die Höhe der Seitenfläche eines Instrumententisches oder der vertikalen Fläche angepasst.

Es ist erfindungsgemäß mindestens eine Fixiermittelzone auf dem Deckel vorgesehen. Weiter vorteilhaft erstreckt sich die Fixiermittelzone im Wesentlichen über die gesamte Länge der Seitenfläche des Einwegbehälters und/oder über die gesamte Länge des Deckels.

Alternativ können auch voneinander beabstandete Fixiermittelzonen vorgesehen sein. Vorzugsweise können auf der mindestens einen Seitenfläche des Einwegbehälters und/oder auf der Unter- und/oder Außenseite des Deckels auch mehrere, also mindestens zwei Fixiermittelzonen vorgesehen sein, welche zueinander durch fixiermittelzonenfreie Bereiche beabstandet sind. Vorzugsweise sind jeweils die Fixiermittelzonen innerhalb der Seitenfläche und/oder innerhalb des Deckels in einer Linie zueinander beabstandet angeordnet.

Insbesondere vorteilhaft können Fixiermittel sowohl im Bereich des Deckels als auch im Bereich einer Seitenfläche des Einwegbehälters angeordnet sein. Auf diese Weise kann alternativ oder zusätzlich eine Festlegung des Einwegbehälters über das Fixiermittel im Bereich des Deckels auf einer horizontalen Fläche insbesondere eines Instrumententisches als auch über das Fixiermittel im Bereich der Seitenfläche des Einwegbehälters an einer vertikalen Fläche, insbesondere einer vertikalen Seitenkante eines Instrumententisches erfolgen.
Die Anwendung der einzelnen Fixiermittel kann damit auf die vorhandenen vertikalen und/oder horizontalen Flächen, insbesondere der eines Instrumententisches abgestimmt werden.

Als Fixiermittel kommen klebende Fixiermittel, wie ein Kleberauftrag, insbesondere ein adhäsiver Kleber oder ein ein- oder zweiseitig klebendes Material, wie beispielsweise eine doppelseitig klebende Folie in Frage, die auf der einen Seite im Bereich der Fixiermittelzone auf dem Einwegbehälter auf der Seitenfläche und/oder auf dem Deckel festgelegt ist und deren andere Seite im unbefestigten Zustand durch ein Releasepaper oder Trennpapier überdeckt sein kann, das dann vor Anwendung und Befestigung an einer horizontalen oder vertikalen Fläche entfernt wird, um so eine klebende Seite freizugeben.

Vorzugsweise können insbesondere Vliesmaterialien als Trägermaterialien, insbesondere für einseitige klebende Materialien, so mit einem darauf insbesondere einseitig beaufschlagten Kleberauftrag, eingesetzt werden. Dies ist insbesondere vorteilhaft im Falle von Instrumententischen, welche mit einer insbesondere Vliesmaterial umfassenden Einmal-Operationsabdeckung bezogen sind, weil damit die durch die Festlegung des Fixiermittels abgedeckten Flächen der Einmal-Operationsabdeckung weiterhin eine Oberseite mit Vliesmaterial, insbesondere eines absorbierenden Vliesmaterials aufweisen.

Alternativ oder zusätzlich können auch Haken- und/oder Ösenhaftmittel vorgesehen sein, insbesondere Hakenhaftmaterialien, die beispielsweise mit einer Abdeckung, die zum Abdecken eines Instrumententisches verwendet wird, und welche vorzugsweise Vliesmaterial umfasst, haftend verbindbar sein können.

Haken-/Ösenhaftmittel werden auch als Hook-/Loop-Elemente bzw. als Kletthaken-/Klettflausch-Elemente bezeichnet.

In jedem Fall ist das Fixiermittel so zu wählen, dass es mit der entsprechenden Oberfläche, insbesondere mit einer Oberfläche von Operations-Einmalabdeckungen, insbesondere mit einer textilen Oberfläche von Operations-Einmalabdeckungen, die für den Instrumententisch vorzugsweise verwendet werden, fixiert werden kann und über die Dauer einer Operation fixiert bleibt.

Insbesondere kann vorgesehen sein, dass im Falle von Fixiermitteln an der Seitenfläche des Einwegbehälters und des Deckels unterschiedliche Fixiermittel, insbesondere eine Kombination aus klebenden Fixiermitteln und aus Ösen - und/oder Hakenhaftelementen eingesetzt wird.

Dabei kann vorgesehen sein, dass der Einwegbehälter nach der Operation nicht wieder von einem Bezug bzw. einer Abdeckung des Instrumententisches entfernt wird, sondern zusammen damit entsorgt werden kann.

Insbesondere kann auch vorgesehen sein, dass der Einwegbehälter nach Entnahme sämtlicher Inhaltsmaterialien zur Aufnahme gebrauchter Materialien verwendet wird, die dann zusammen mit dem Einwegbehälter entsorgt werden können.

Des Weiteren betrifft die Erfindung ein wegwerfbares OP-Spendersystem, wobei unter der Abkürzung OP stets Operation verstanden werden soll. Dieses wegwerfbare OP-Spendersystem umfasst einen Einwegbehälter der vorstehend beschriebenen Art, wobei im Innenraum herstellerseitig sterile, absorbierende Verbandstoffe vorgelegt sind. Diese wegwerfbaren OP-Spendersysteme sind vorteilhafterweise, insbesondere in einer weiteren Umverpackung, steril verpackt. Zur Sterilisierung kommt insbesondere eine Dampfsterilisierung in Frage. Insbesondere betrifft die Erfindung ein steriles wegwerfbares OP-Spendersystem.

Die Erfindung wird im Folgenden anhand einer Zeichnung näher erläutert. Dabei zeigen:
- Figur 1: einen Instrumententisch mit daran angebrachtem Einwegbehälter;
- Figur 2: einen erfindungsgemäßen Einwegbehälter;
- Figur 3: verschiedene Gestaltungen der Fixiermittelzone am Einwegbehälter;
- Figur 4: einen Instrumententisch mit daran angebrachtem Einwegbehälter;
- Figur 5: eine weitere Ausgestaltung einer Anbringung eines erfindungsgemäßen Einwegbehälters an einem Instrumententisch;
- Figur 6: alternative Anbringungen der Fixiermittelzone am Einwegbehälter und
- Figur 7: ein an einem Instrumententisch angeordneter Einwegbehälter.

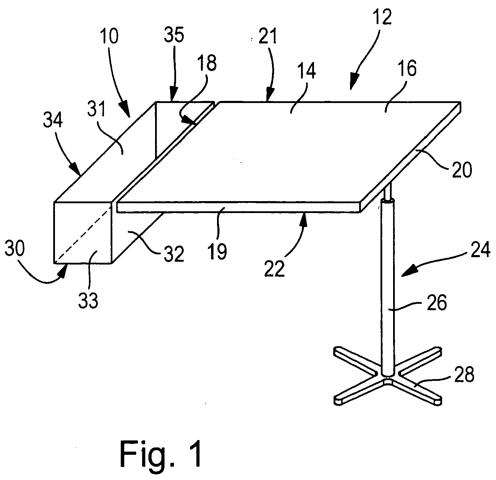

Figur 1 zeigt einen Instrumententisch, der mit dem Bezugszeichen 12 versehen ist, wobei der Instrumententisch aus einer Tischplatte 14 mit einer horizontalen nach oben gerichteten Fläche 16 sowie vier Seitenflächen 18, 19, 20 und 21 sowie einer Unterseite 22 ausgebildet ist. Der Instrumententisch 12 weist darüber hinaus einen Fuß 24 auf bestehend aus einem Bein 26 sowie einem kreuzförmig gestalteten Stativ 28.

An einer der Seitenflächen, hier an der Seitenfläche 18, ist ein Einwegbehälter 10 zum Darbieten von Verbandstoffen in einem Operationssaal gezeigt mit einer Bodenfläche 30 sowie mindestens einer Seitenfläche 32, wobei bei dem hier gezeigten quaderförmigen Einwegbehälter vier Seitenflächen, nämlich 32, 33, 34 und 35, vorgesehen sind, die gemeinsam mit der Bodenfläche 30 einen Innenraum 31 begrenzen, wobei in dem Innenraum Verbandstoffe herstellerseitig vorgelegt und dargeboten werden können. Die Seitenflächen 32 bis 35 sind mit jeweils einer unteren Seitenkante mit der Bodenfläche 30 verbunden.

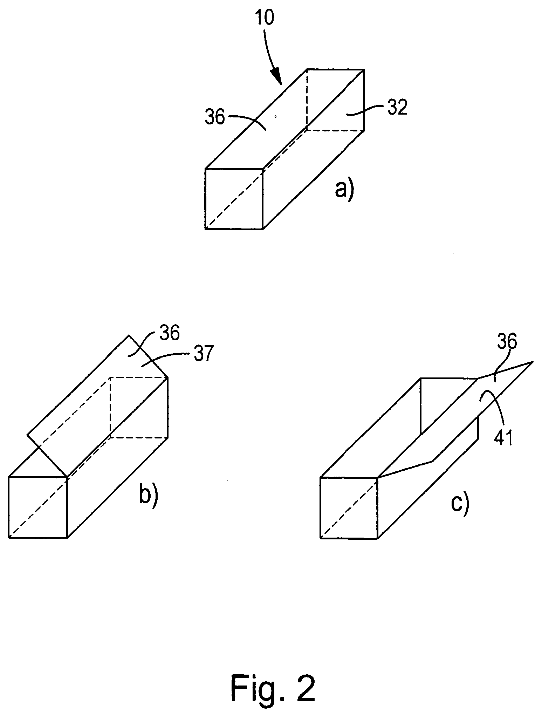

Erfindungsgemäß ist bei dem Einwegbehälter 10 ein Deckel vorgesehen, welcher wie in der Figur 2 dargestellt, fest mit dem Einwegbehälter 10 verbunden ist.

Figur 2 zeigt nun in drei Darstellungen den Einwegbehälter 10, wobei in der Darstellung a) der Einwegbehälter mit geschlossenem Deckel 36 zu sehen ist. Der Deckel 36 ist insbesondere an einer oberen Seitenkante, hier der Seitenfläche 32, angelenkt und kann von der gezeigten geschlossenen Position in eine geöffnete Position verschwenkt werden. In den Darstellungen b) und c) wird der Deckel 36 dann in verschiedenen Öffnungsstellungen gezeigt. Der Deckel weist dabei eine Unterseite bzw. Innenseite 41 und eine Oberseite bzw. Außenseite 37 auf, was also bei geschlossenem Deckel der auf den Innenraum hin gerichteten Seite und der davon abgewandten Seite entspricht.

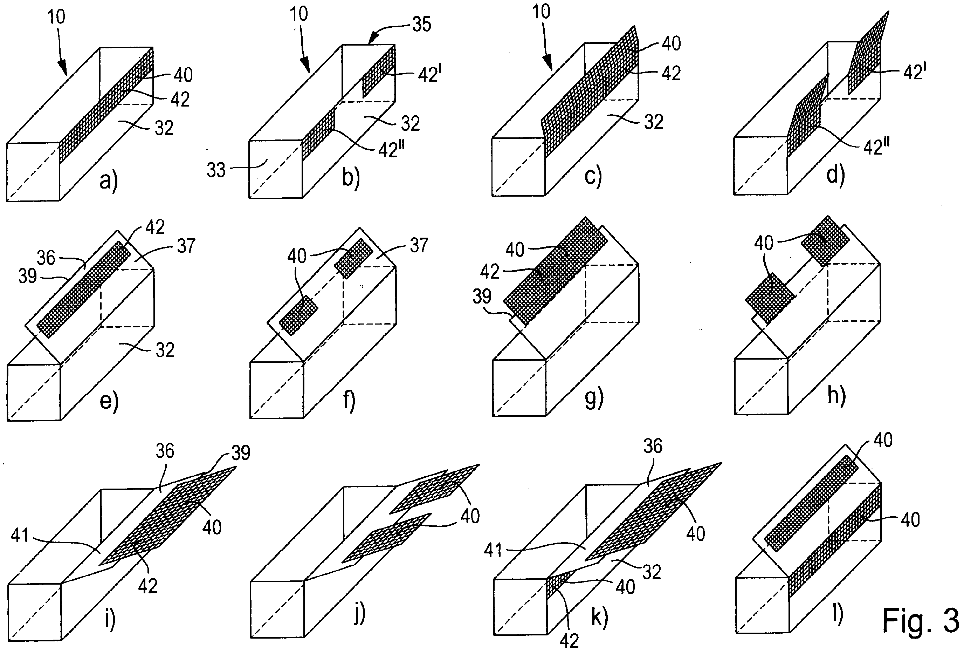

Figur 3 zeigt eine Vielzahl von Einwegbehältern, wobei die Darstellungen mit a) bis l) bezeichnet sind. Die Darstellungen a) - d) zeigen nicht erfindungsgemäße Einwegbehälter ohne Deckel.

Zur Anbringung des Einwegbehälters 10 am Instrumententisch 12 sind Fixiermittel 40 vorgesehen, die im Bereich einer Fixiermittelzone 42 am Einwegbehälter 10 angebracht sind. Die Fixiermittel 40 können dabei ausschließlich in der Fixiermittelzone befindlich sein oder sich über die Fixiermittelzone 42 hinaus erstrecken. Als Fixiermittel kommt beispielsweise ein adhäsiver Kleberauftrag oder auch ein doppel- oder einseitiges Klebeband in Frage, das im Falle eines einseitigen Klebebandes beispielsweise adhäsiv über einen zusätzlichen Kleber im Bereich der Fixiermittelzone 42 an den Einwegbehälter 10 angebracht sein kann.

Darstellung a) zeigt eine erste Gestaltung, wie sie auch in Figur 1 Verwendung findet, wobei im Bereich der oberen Seitenkante der Seitenfläche 32 eine Fixiermittelzone 42 vorgesehen ist, die sich bei dem quaderförmigen Einwegbehälter 10 im Wesentlichen über die gesamte Länge der Seitenfläche 32 erstreckt und hinsichtlich ihrer Höhe an die Höhe der Seitenfläche 18 des Instrumententisches 12 angepasst sein kann. Sofern der Instrumententisch 12 mit einer Abdeckung beispielsweise aus einem Vliesstoff abgedeckt ist, kann jedoch die Fixiermittelzone 42 auch eine größere Erstreckung in der Höhe aufweisen und auch in diesem Bereich noch mit der Abdeckung des Instrumententisches 12 verankert sein. Als Fixiermittel 40 ist hier ein doppelseitiges Klebeband vorgesehen, das mit seiner einen Seite an der Seitenfläche 32 des Einwegbehälters 10 angeheftet ist und dessen andere Seite vor Verwendung zunächst mit einem Trennpapier oder Releasepaper überdeckt ist, um ein unerwünschtes Anhaften vor Verwendung zu vermeiden. Soll der Einwegbehälter 10 dann beispielsweise an einem Instrumententisch angebracht werden, kann vorher das Releasepaper entfernt werden und die zweite klebende Seite des Fixiermittels 40 wird freigelegt.

Darstellung b) zeigt einen Einwegbehälter 10 mit zwei Fixiermittelzonen 42' und 42'', die ebenfalls an der Seitenfläche 32 im Bereich der oberen Seitenkante angeordnet sind, und zwar jeweils angrenzend zu den Seitenflächen 35 und 33 des Einwegbehälters. Zwischen den beiden Fixiermittelzonen 42' und 42'' ist ein fixiermittelfreier Bereich vorgesehen.

Darstellung c) zeigt einen Einwegbehälter 10 mit einer Fixiermittelzone 42, die der von Darstellung a) entspricht. Hier ist allerdings das Fixiermittel 40 nicht nur im Bereich der Fixiermittelzone 42 angebracht, sondern erstreckt sich über die obere Seitenkante der Seite 32 hinaus. Dies bietet den Vorteil, dass neben einer Anbringung an der Seitenfläche 18 des Instrumententisches das Fixiermittel noch umgelegt werden kann und so auf der horizontalen Fläche 16 des Tisches befestigt werden kann. Hierdurch wird eine noch sicherere Befestigung erreicht.

Darstellung d) zeigt eine analoge Gestaltung zu Darstellung c), jedoch hier mit zwei Fixiermittelzonen 42' und 42'' analog zu Darstellung b).

Darstellung e) zeigt eine Gestaltung, bei der die Fixiermittelzone 42 im Bereich des Deckels 36, der halb geöffnet dargestellt ist, angebracht ist, und zwar auf der Außenseite 37 des Deckels. Die Fixiermittelzone 42 erstreckt sich dabei über einen wesentlichen Teil der Länge des Deckels und ist ausgehend von der Seitenkante mit der Seitenfläche 32 eher in Richtung der freien Seitenkante 39 des Deckels angeordnet.

Darstellung f) zeigt eine Anbringung eines Fixiermittels 40, das hierum wiederum auf zwei Fixiermittelzonen 42 aufgeteilt ist, aber ebenfalls auf der Außenseite 37 des Deckels 36 angeordnet ist.

In Darstellung g) ist ebenfalls eine Anordnung des Fixiermittels 40 im Bereich des Deckels, und zwar hier im Bereich der freien Seitenkante 39 des Deckels vorgesehen, wobei sich das Fixiermittel 40 zum Teil im Bereich der Fixiermittelzone 42, zum Teil jedoch auch außerhalb dieser über die Kante 39 hinaus erstreckt.

Darstellung h) zeigt wiederum eine analoge Gestaltung zur Darstellung g), wobei hier zwei Fixiermittel 40 beabstandet voneinander vorgesehen sind.

Darstellung i) zeigt eine Gestaltung entsprechend Darstellung g), wobei hier jedoch das Fixiermittel 40, das wiederum über die freie Kante 39 des Deckels festgelegt ist und die Fixiermittelzone 42 im Bereich der freien Kante 39 der Deckelinnenseite 41 vorgesehen ist.

Darstellung j) zeigt wiederum eine analoge Gestaltung, hier jedoch mit zwei Fixiermitteln 40, die voneinander beabstandet, ansonsten aber analog zur Anordnung gemäß Darstellung i) vorgesehen sind.

In Darstellung k) ist zunächst ein Fixiermittel, wie bei Darstellung i) auf der Innenseite 41 des Deckels 36 angeordnet. Darüber hinaus ist ein weiteres Fixiermittel 40 im Bereich einer Fixiermittelzone 42, wie bei Darstellung a), an der oberen Seitenkante der Seitenfläche 42 vorgesehen. Auf diese Weise kann alternativ eine Festlegung über das Fixiermittel im Bereich des Deckels 36 erfolgen, wobei hier vorgesehen sein kann, dass nicht die in der Darstellung nach oben weisende Seite des Fixiermittels 40 im Bereich des Deckels klebend ausgebildet ist, sondern die in der Darstellung nach unten weisende Seite, die bei einem aufgeklappten Deckel 36 auf eine horizontale Fläche 16 eines Instrumententisches 12 aufgelegt werden könnte. Zusätzlich kann dann ein entsprechender Einwegbehälter 10 noch über die Fixiermittel 40 im Bereich der Seitenfläche 32 an der Seitenkante 18 des Tisches befestigt werden.
Darstellung l) zeigt nun eine Gestaltung, bei der das Fixiermittel 42 zum einen wie in Darstellung e) und zum anderen wie in Darstellung a) angeordnet ist. Auch hierdurch wird eine doppelte Befestigung zum einen im Bereich der horizontalen Fläche 16 und zum anderen im Bereich der Seitenfläche 18 des Instrumententisches 12 ermöglicht.

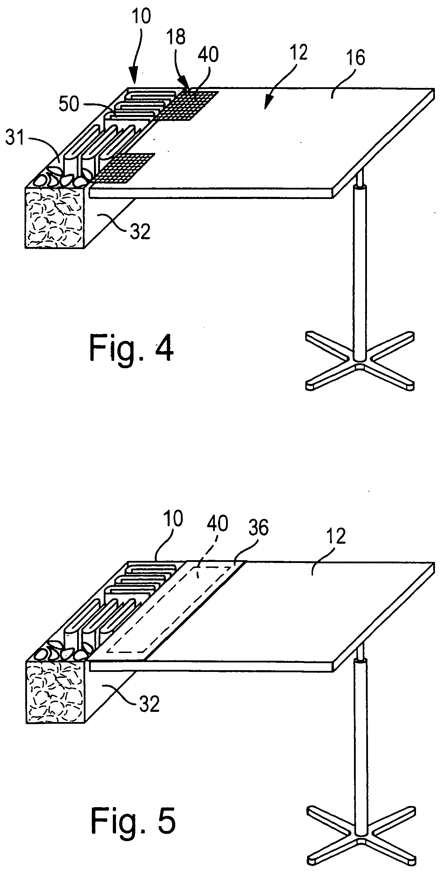

Figur 4 zeigt nun eine nicht erfindungsgemäße Ausgestaltung mit einem Instrumententisch 12, der analog Figur 1 ausgestaltet ist, sowie einen hieran angebrachten Einwegbehälter 10, der wiederum als Faltschachtel ausgebildet ist und aus einem Pappmaterial besteht. Gut zu erkennen ist hierbei die Anordnung von Verbandstoffen 50 im Innenraum 31 des Einwegbehälters 10. Die Verbandstoffe 50 können dabei insbesondere mit ihren Faltkanten in der Darstellung nach oben, ausgerichtet sein, da dies ein einfacheres Zählen der Absorptions- und Verbandmaterialien durch das Operationspersonal ermöglicht. Darüber hinaus weist der Einwegbehälter 10 zwei Fixiermittel 40 auf, die analog zur Darstellung gemäß Figur 3d ausgestaltet sind und eine Fixierung sowohl im Bereich der Seitenkante 18 des Tisches als auch der horizontalen Fläche 16 des Instrumententisches 12 ermöglichen. Dabei kann der Instrumententisch 12 mit einer (hier nicht dargestellten) Operationsabdeckung versehen sein, die insbesondere aus einem Vliesstoff gebildet ist und die Fixiermittel 40 sind so ausgebildet, dass sie sicher an dem Vliesmaterial der Operationsabdeckung haften. Dies besitzt den Vorteil, dass am Ende die Operationsabdeckung des Instrumententisches 12 zusammen mit dem Einwegbehälter 10, der zu diesem Zeitpunkt darüber hinaus auch mit verbrauchten Verbandmaterialien gefüllt sein kann, entsorgt werden kann. Der Deckel 36 wurde hier vollständig entfernt, wozu beispielsweise vorgesehen sein kann, dass entlang der oberen Seitenkante der Seitenfläche 32 eine Perforation vorgesehen sein kann, entlang der der Deckel 36 von der Seitenfläche 32 getrennt werden kann.

Figur 5 zeigt nun eine erfindungsgemäße Gestaltung, wobei hier jedoch der Deckel 36 am Einwegbehälter 10 verblieben ist und der Deckel 36 aus seiner geschlossenen Position um 180° verschwenkt worden ist, wobei der Deckel 36 über seine gesamte Länge mit der Seitenfläche 32 gelenkig verbunden ist. Die Anbringung des Fixiermittels in einer Fixiermittelzone 42 erfolgt hierbei wie in Figur 3e dargestellt auf der Außenseite 37 des Deckels 36 in Form eines doppelseitigen Klebebandes. Zusätzlich kann noch wie in Darstellung l) von Figur 3 ein weiteres Fixiermittel 40 an der oberen Seitenkante der Seitenfläche 32 vorgesehen sein, was hier jedoch durch den Instrumententisch 12 verdeckt ist.

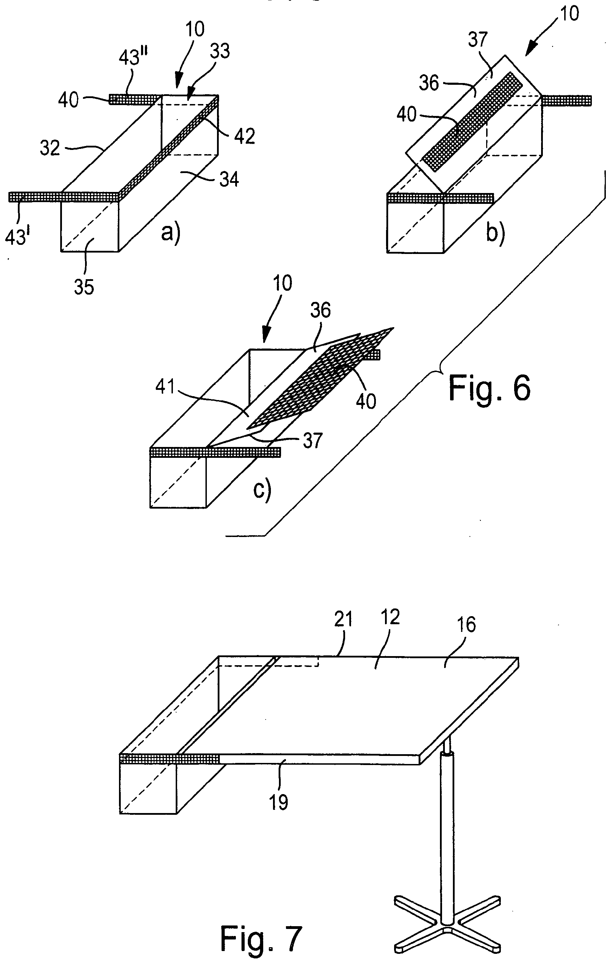

Weitere alternative Befestigungsmöglichkeiten zeigen nun die Darstellungen a) bis c) in Figur 6 zur Festlegung eines Einwegbehälters 10 an einem Instrumententisch. So zeigt Darstellung a) eine Vorsehung einer Fixiermittelzone 42, die sich sowohl über die obere Seitenkante der Fläche 34 als auch die oberen Seitenkanten der beiden Seitenflächen 35 und 33 erstreckt. Darüber hinaus ist vorgesehen, dass das Fixiermittel 40 sich über die Seitenflächen 33 und 35 und deren Kante, mit der diese beiden Seitenflächen 33, 35 mit der Seitenfläche 32 verbunden sind, hinaus erstreckt, so dass zum einen über drei Seiten umlaufend das Fixiermittel 40 vorgesehen ist und so eine sehr gute Verankerung am Einwegbehälter gewährleistet und darüber hinaus zwei Abschnitte 43' und 43'' des Fixiermittels 40 sich über den Einwegbehälter hinaus erstrecken. Besonders vorteilhaft ist dabei, dass das Fixiermittel 40 in Form eines durchgehenden Klebebandes vorgesehen sein kann, das einstückig über die gesamte Länge ausgebildet sein kann. Das Fixiermittel 40 wird dann im Bereich der überstehenden Bereiche 43' und 43'' an einem Instrumententisch, wie in Figur 7 gezeigt, festgelegt, und zwar dort nicht im Bereich der Seitenfläche 18, sondern im Bereich der Flächen 19 und 21.

Darstellung b) zeigt eine erfindungsgemäße Gestaltung, wobei hier zusätzlich noch auf der Deckelaußenseite 37 des Deckels 36 ein Fixiermittel 40 vorgesehen ist, so dass eine zusätzliche Befestigung auf der horizontalen Fläche 16 des Instrumententisches bereitgestellt werden kann.

Darstellung c) zeigt wiederum eine erste Fixiermittelanordnung gemäß Darstellung 6 a) und zusätzlich erfindungsgemäß ein Fixiermittel 40 auf der Innenfläche 41 des Deckels 36, das über die freie Kante des Deckels 36 hinaus reicht, so dass hierüber ebenfalls eine Fixierung auf der horizontalen Fläche 16 des Instrumententisches 12 erfolgen kann, indem die zur Oberseite 37 des Deckels 36 gewandte Fläche des Fixiermittels 40 klebend ausgebildet ist und am Instrumententisch fixiert werden kann.

Auf die vorstehend beschriebene Weise kann besonders einfach eine Fixierung eines wegwerfbaren Einwegbehälters 10 an einem Instrumententisch 12 bereitgestellt werden, wobei der Platz auf der horizontalen Fläche 16 des Instrumententisches nicht zum Abstellen eines Einwegbehälters für Verbandstoffe benötigt wird, so dass durch die Fixierung des Einwegbehälters 10 an den Flächen des Instrumententisches 12 ein zusätzliches Platzangebot bereitgestellt werden kann, ohne dass auf eine wiederverwertbare Gestaltung, die manuell befüllt werden muss und zu sterilisieren ist, zurückgegriffen werden muss.

## Patentansprüche

1. Einwegbehälter zum Darbieten von Verbandsstoffen im Operationssaal, mit einer Bodenfläche (30) sowie mindestens einer Seitenfläche (32-35), die zusammen mit der Bodenfläche (30) einen Innenraum (31) begrenzt, wobei die mindestens eine Seitenfläche (32-35) mit einer unteren Seitenkante mit der Bodenfläche (30) verbunden ist, sowie einem Deckel (36), der an einer der Seitenflächen (32-35) an einer oberen Seitenkante öffenbar angelenkt ist, **dadurch gekennzeichnet, dass** mindestens ein Bereich des Deckels (36) als Fixiermittelzone (42) mit einem Fixiermittel (40) ausgebildet ist, über das der Einwegbehälter (10) an einer vertikalen (18-21) und/oder horizontalen Fläche (16), insbesondere eines Instrumententischs (12), fixierbar ist.

2. Einwegbehälter nach Anspruch 1, wobei der Einwegbehälter (10) quaderförmig ist und vier, insbesondere gerade Seitenflächen (32-35) aufweist.

3. Einwegbehälter nach Anspruch 1 oder 2, wobei ein Bereich mindestens einer der Seitenflächen (32-35) als Fixiermittelzone (42) mit einem Fixiermittel (40) ausgebildet ist, über das der Einwegbehälter (10) an einer vertikalen (18-21) und/oder horizontalen Fläche (16), insbesondere eines Instrumententisches (12), fixiert ist.

4. Einwegbehälter nach einem der Ansprüche 1 bis 3, wobei das Fixiermittel (40) sich über die Fixiermittelzone (42) der mindestens einen Seitenfläche (32-35) und/oder des Deckels (36) hinaus erstreckt.

5. Einwegbehälter nach einem der vorangehenden Ansprüche, wobei die Fixiermittelzone (42) auf der Innen- (41) und/oder Außenseite (37) des Deckels (36) angebracht ist.

6. Einwegbehälter nach einem der vorangehenden Ansprüche, wobei das Fixiermittel (40) durch einen Klebeauftrag, ein ein- oder zweiseitig klebendes Material und/oder ein Haken- und/oder Ösen-Haftmaterial gebildet ist, wobei das Fixiermittel (40) insbesondere durch ein entfernbares Abdeckpapier abdeckbar ausgebildet ist.

7. Wegwerfbares OP-Spendersystem umfassend einen Einwegbehälter nach einem der vorangehenden Ansprüche, wobei im Innenraum herstellerseitig sterile, absorbierende Verbandstoffe vorgelegt sind.

8. Wegwerfbares OP-Spendersystem nach Anspruch 7, wobei das System steril verpackt ist.

## Claims

1. A disposable container for presenting dressings in the operating room, having a bottom face (30) and at least one side face (32-35), which together with the bottom face (30) defines an interior (31), the at least one side face (32-35) being connected by a lower side edge to the bottom face (30), and having a lid (36), which is pivotably connected in openable fashion on one of the side faces (32-35) to an upper side edge, **characterized in that** at least one region of the lid (36) is embodied as a fixation-means zone (42) having a fixation means (40) by way of which the disposable container (10) is fixable to a vertical face (18-21) and/or horizontal face (16), in particular of an instrument table (12).

2. The disposable container of claim 1, wherein the disposable container (10) is cuboid and has four side faces (32-35) that are in particular rectilinear.

3. The disposable container of claim 1 or 2, wherein a region of at least one of the side faces (32-35) is embodied as a fixation-means zone (42) having a fixation means (40) by way of which the disposable container (10) is fixed to a vertical face (18-21) and/or horizontal face (16), in particular of an instrument table (12).

4. The disposable container of one of claims 1 through 3, wherein the fixation means (40) extends outward via the fixation-means zone (42) of the at least one side face (32-35) and/or of the lid (36).

5. The disposable container of one of the foregoing claims, wherein the fixation-means zone (42) is attached to the inner side (41) and/or outer side (37) of the lid (36).

6. The disposable container of one of the foregoing claims, wherein the fixation means (40) is formed by an application of glue, a material that is adhesive on one or two sides, and/or a hook and/or eye self-adhesive material, and the fixation means (40) is embodied in particular as capable of being exposed by means of a removable backing paper.

7. A disposable operating room dispenser system, including a disposable container of one of the foregoing claims, wherein sterile, absorbent dressings are placed inside the interior in advance by the manufacturer.

8. The disposable operating room dispenser system of claim 7, wherein the system is packaged in sterile fashion.

## Revendications

1. Contenant jetable permettant de mettre à disposition des pansements en salle d'opération, comprenant une surface inférieure (30) ainsi qu'au moins une surface latérale (32-35) qui délimite conjointement avec la surface inférieure (30) un espace intérieur (31), la ou les surfaces latérales (32-35) étant reliées par un bord latéral inférieur à la surface inférieure (30), ainsi qu'un couvercle (36) qui est articulé sur une des surfaces latérales (32-35) de manière à pouvoir être ouvert au niveau d'un bord latéral supérieur, **caractérisé en ce qu'**au moins une zone du couvercle (36) est réalisée sous la forme d'une zone (42) de moyen de fixation présentant un moyen de fixation (40) par l'intermédiaire duquel le contenant jetable (10) peut être fixé sur une surface verticale (18-21) et/ou horizontale (16), en particulier d'une table à instruments (12).

2. Contenant jetable selon la revendication 1, le contenant jetable (10) étant parallélépipédique et comprenant quatre surfaces latérales (32-35), en particulier rectilignes.

3. Contenant jetable selon la revendication 1 ou 2, une zone d'au moins une des surfaces latérales (32-35) étant réalisée sous la forme d'une zone (42) de moyen de fixation pourvue d'un moyen de fixation (40) par l'intermédiaire duquel le contenant jetable (10) est fixé sur une surface verticale (18-21) et/ou horizontale (16), en particulier d'une table à instruments (12).

4. Contenant jetable selon l'une quelconque des revendications 1 à 3, le moyen de fixation (40) s'étendant au-delà de la zone (42) de moyen de fixation de la ou des surfaces latérales (32-35) et/ou du couvercle (36).

5. Contenant jetable selon l'une quelconque des revendications précédentes, la zone (42) de moyen de fixation étant montée sur la face intérieure (41) et/ou la face extérieure (37) du couvercle (36).

6. Contenant jetable selon l'une quelconque des revendications précédentes, le moyen de fixation (40) étant formé par une couche de colle, un matériau adhésif une face ou deux faces et/ou un matériau adhésif à crochets et/ou à oeillets, le moyen de fixation (40) pouvant en particulier être recouvert par un papier couvrant amovible.

7. Système de distribution jetable pour salle d'opération comprenant un contenant jetable selon l'une quelconque des revendications précédentes, des pansements absorbants stériles étant mis à disposition par le fabricant dans l'espace intérieur.

8. Système de distribution jetable pour salle d'opération selon la revendication 7, le système étant emballé de manière stérile.
